# EUROPÄISCHE PATENTSCHRIFT

(11) **EP 1 774 979 B1**
(45) Veröffentlichungstag und Bekanntmachung des Hinweises auf die Patenterteilung: **14.10.2009**
(21) Anmeldenummer: 06017369.7
(22) Anmeldetag: 21.08.2006
(51) Int. Cl.: A61L 2/025, A47L 15/23

(54) **Einrichtung zur Reinigung von medizinischen Instrumenten mittels Ultraschall**
Ultrasonic sanitising device for medical instruments
Appareil de nettoyage à ultrasons pour des instruments médicaux

(30) Priorität: 11.10.2005 DE 102005049005
(43) Veröffentlichungstag der Anmeldung: 18.04.2007
(73) Patentinhaber: Vanguard AG, 10117 Berlin (DE)
(72) Erfinder: Schrödel, Robert, 14193 Berlin (DE)
(74) Vertreter: Haft, von Puttkamer, Berngruber, Karakatsanis

(56) Entgegenhaltungen:
- EP-A2- 1 073 095
- DE-A1- 19 858 347
- US-A1- 2003 010 356

## Beschreibung

Die vorliegende Erfindung betrifft eine Einrichtung zur Reinigung von medizinischen Instrumenten mittels Ultraschall nach dem Oberbegriff des Patentanspruches 1.

Eine Einrichtung dieses Art geht aus des US 2003/010356 A1 hervor.

Medizinische Instrumente werden übliches Weise in herkömmlichen Ultraschallbädern gereinigt. Dabei werden in einem Behälter, der mit einer Übertragungsflüssigkeit, beispielsweise Wasser gefüllt ist, mit der Hilfe von sogenannten Ultraschallgeneratoren, die beispielsweise am Boden des Behälters angeordnet sind, Ultraschallwellen erzeugt, die sich im Wasser ausbreiten, das als Vorlaufstrecke dient. Die Frequenz der von den Ultraschallgeneratoren erzeugten Ultraschallwellen liegt in einem Bereich von 30kHz bis 2 GHz. Die Frequenz wird in Abhängigkeit von der kleinsten Struktur des zu reinigenden medizinischen Instrumentes ausgewählt. Die derzeit übliche Frequenz beträgt für Partikel mit einem Durchmesser > 1µm ca. 200 kHz. Bei 400 kHz bis 1-2 MHz werden Partikel mit einem Durchmesser < 1µm optimal gereinigt.

In der Übertragungsflüssigkeit entstehen insbesondere durch Interferenzen der Ultraschallwellen in an sich bekannter Weise Druckschwankungen in der Form von sich abwechselnden Über- und Unterdrücken, wobei beim Unterschreiten des Dampfdruckes der Übertragungflüssigkeit Dampfbläschen erzeugt werden, die danach wieder kollabieren. Bei der Implosion der Dampfbläschen entstehen sogenannte Mikrojets in der Form von kleinen Wasserstrahlen, die sich bevorzugt in Richtung angrenzender Oberflächen ausdehnen und an dieser hohe Drücke bewirken, die zu einer mechanischen Abtragung von Kontaminationen der Oberflächen der zu reinigenden Instrumente führen.

Eine nach diesem Prinzip arbeitende Ultraschall-Waschmaschine geht aus der DE 39 24 519 A1 hervor. Diese Waschmaschine ist unter anderem auch für gewerbliche und industrielle Anwendungen geeignet.

Aus der DE 34 28 540 C2 geht eine Vorrichtung zur Erzeugung einer Kavitation in einer strömenden Flüssigkeit hervor, wobei ein Hochbehälter einen vorzugsweise horizontal verlaufenden Auslaufstutzen, wenigstens einen an den horizontalen Auslaufstutzen anschließenden Rohrkrümmer und ein vertikales Fallrohr mit einer Regelarmatur aufweist. In dem Fallrohr wird eine Kavitationsstrecke dadurch gebildet, dass nach dem Rohrkrümmer Abtrenneinrichtungen zur Entnahme bzw. Abtrennung des kavitierten Strömungsanteiles von der Hauptströmung vorgesehen sind, wobei sich der Durchmesser des Fallrohres nach der Abtrenneinrichtung vergrößert.

In der DE 198 58 347 A1 ist ein Verfahren zum Reinigen, chemischen Sterilisieren oder Desinfizieren von medizinischen Geräten erläutert, bei dem ein Gerät in einem Behälter mit einer Reinigungslösung gereinigt und danach mit einer Spüllösung gespült sowie schließlich mit einem flüssigen Sterilisierungsmittel behandelt wird. Das Sterilisierungsmittel wird verdampft, wobei das Gerät gleichzeitig sterilisiert und getrocknet wird. Dabei ist es denkbar, in dem Behälter ober- und/oder unterseitig einen oder mehrere Rührer anzuordnen, die die Form von rotierenden Dreharmen aufweisen. Die Dreharme können hohl ausgeführt sein oder können Kanäle besitzen, die mit einer außen angeordneten Quelle für die Übertragungsflüssigkeit verbunden sind.

Dabei können an einer Wandung des Behälters Ultraschallgeneratoren vorgesehen sein.

Die Aufgabe der vorliegenden Erfindung besteht darin, eine Einrichtung zum Reinigen von Gegenständen, insbesondere von medizinischen Gegenständen dahingehend zu verbessern, dass bei möglichst geringem Wasserverbrauch eine optimale Reinigungswirkung an der Oberfläche der Instrumente erreicht wird.

Diese Aufgabe wird durch eine Einrichtung mit den Merkmalen des Patentanspruches 1 gelöst.

Der wesentliche Vorteil der vorliegenden Erfindung besteht darin, dass eine insbesondere großflächige Reinigung der Flächen von medizinischen Instrumenten, insbesondere auch von demontierten Instrumenten, dadurch bei einer guten Reinigungswirkung und bei geringem Wasserverbrauch erzielt wird, dass von zwei hohlen Drehflügeln einer sich drehenden Drehflügelanordnung aus mit einer Ultraschwingung beaufschlagte Flüssigkeitsstrahlen abgegeben und auf die zu reinigenden Flächen der medizinischen Instrumente gerichtet werden. Eine besonders gute Reinigungswirkung ergibt sich dadurch, dass die ultraschallbehafteten Flüssigkeitsstrahlen beim Auftreffen auf die Oberflächen der zu reinigenden Instrumente und die dabei bewirkte Kavitation eine besonders gute Reinigungswirkung entfalten. Dies dürfte insbesondere auch auf Interferenzen zurückzuführen sein, die durch die Überlagerung der Ultraschallwellen der einzelnen Flüssigkeitsstrahlen erzielt werden.

Vorteilhafte Ausgestaltungen der Erfindung gehen aus den Unteransprüchen hervor.

Im Folgenden werden die Erfindung und deren Ausgestaltungen im Zusammenhang mit den Figuren näher erläutert. Es zeigen:
Figur 1 in schematischer Darstellung eine erste Ausführungsform der erfindungsgemäßen Einrichtung, bei der wenigstens ein Ultraschallgenerator in der hohlen Drehachse einer Drehflügelanordnung untergebracht ist, wobei die in der hohlen Drehachse erzeugten Ultraschallwellen über Reflektoren zu den einzelnen Düsen der Drehflügel der Drehflügelanordnung reflektiert werden;
Figur 2 eine zweite Ausführungsform der erfindungsgemäßen Drehflügelanordnung, bei der in den Drehflügeln den einzelnen Düsen zugeordnete Ultraschallgeneratoren vorgesehen sind; und
Figuren 3A, 3B und 4 vorteilhafte Weiterbildungen der Erfindung.

Zu der Erfindung führten die folgenden Überlegungen. Statt der Erzeugung von Ultraschallwellen in einer in einem Behälter enthaltenen Übertragungsflüssigkeit durch Anordnung von Ultraschallgeneratoren innerhalb und/oder außerhalb des Behälters, kann eine wesentlich bessere mechanische Reinigungs- und Abtragungswirkung an den Oberflächen von in dem Behälter angeordneten medizinischen Instrumenten dadurch erreicht werden, dass die entsprechenden Oberflächen der Instrumente durch Flüssigkeitsstrahlen einer Übertragungsflüssigkeit beaufschlagt werden, die von den Düsen der Drehflügel einer Drehflügelanordnung abgegeben werden, wobei die Flüssigkeitsstrahlen die mit Ultraschallwellen beaufschlagt sind.

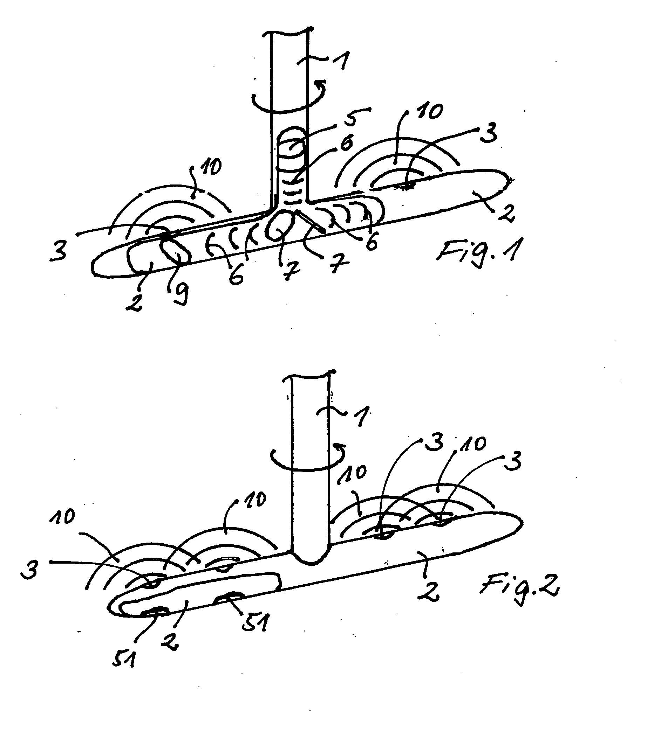

Die Figur 1 zeigt in schematischer Darstellung eine erste Ausführungsform der vorliegenden Drehflügelanordnung, die im Wesentlichen aus einer drehbaren hohlen Drehachse 1 und vorzugsweise zwei daran angeordneten hohlen Drehflügeln 2 besteht. Die Drehflügel 2 weisen vorzugsweise jeweils mehrere Düsen 3 auf, die zu der Seite von in einem Behälter angeordneten medizinischen Instrumente (nicht dargestellt) ausgerichtet sind.

In der hohlen Drehachse 1 ist wenigstens ein Ultraschallgenerator 5 angeordnet, des als Quelle zur Erzeugung von Ultraschallwellen 6 in der die Drehachse 1 durchströmenden Übertragungsflüssigkeit dient. Am Ende des hohlen Schaftteiles 1 sind Reflektoren 7 vorgesehen, die die aus der Drehachse 5 austretenden Ultraschallwellen 6 in die Drehflügel 2 reflektieren bzw. leiten. Die auf diese Weise in den Drehflügel 2 übertragenen Ultraschallwellen 6 breiten sich radial nach außen zu dem Bereich der Düsen 3 aus, wobei sie an weiteren Reflektoren 9 in Richtung auf die Düsen 3 reflektiert werden.

Aus diesem Grunde ist der von den Düsen 3 abgegebene Flüssigkeitsstrahl 10 des über die Drehachse 1 zugeführten flüssigen Übertragungsmediums von Ultraschallwellen beaufschlagt. Besonders bevorzugt handelt es sich bei dem flüssigen Übertragungsmedium um Wasser, wobei darauf geachtet werden muss, dass sich die materialspezifische Schallgeschwindigkeit und Dichte von den Parametern des umgebenden Fluids unterscheiden. Als Reflektoren 7 und 9 werden vorzugsweise in den Drehflügeln 2 schräg montierte Reflektorelemente bzw. -spiegel verwendet.

Die Drehflügel 2 liegen sich an einem Ort des hohlen Drehaschse 1 des Drehflügelanordnung radial gegenüber.

Die Figur 2 zeigt eine zweite bevorzugte Ausführungsform der Erfindung, bei der das flüssige Übertragungsmedium wieder über die hohle Drehachse 1 zu den hohlen Drehflügeln 2 zugeführt wird und über die Düsen 3 desselben strahlartig ausströmt. Im Unterschied zu der ersten Ausführungsform der Figur 1 ist in den hohlen Drehflügeln 2 jeder Düse 3 ein Ultraschallgenerator 51 zugeordnet, des jeweils in dem zu und durch die Düsen 3 strömende Übertragungsmedium Ultraschallwellen erzeugt. Da an den einzelnen Drehflügeln 2 nebeneinander mehrere Düsen 3 angeordnet sind, kommt es zu einer besonders vorteilhaften Überlagerung der Ultraschallwellen der aus den Düsen 3 austretenden Flüssigkeitsstrahlen 10, so dass Interferenzen gebildet werden, die zu einer besonders vorteilhaften Kavitationsbildung führen, wie dies eingangs bereits erwähnt wurde.

Es wird darauf hingewiesen, dass die Drehflügelanordnung des vorliegenden Einrichtung an der Stelle der erläuterten beiden, sich radial gegenüberliegenden Drehflügel 2 auch mehrere Drehflügel aufweisen kann.

Es ist denkbar die Flüssigkeitsstrahlen direkt auf die zu reinigenden Instrumente aufzusprühen oder alternativ die Instrumente und die Drehflügelanordnung in einem Behälter mit einer Übertragungsflüssigkeit anzuordnen und die Flüssigkeitsstrahlen der Drehflügel in diese Übertragungsflüssigkeit einzukoppeln. Dabei wird der Erhalt des Flüssigkeitsstrahls durch einen drallförmigen Impuls, d.h. also durch eine Drehung des Flüssigkeitsstrahls gewährleistet. Dies bedeutet, dass der Flüssigkeitsstrahl 10 durch einen geeigneten schnecken- oder wendelförmigen Auslauf der Düse 3 eine Drehung erfährt.

Zur Erzielung einer gezielten Reinigung können die erwähnten Drehflügel in unterschiedlichen Weisen angeordnet werden. Beispielsweise können in einem Behälter mehrere Drehflügel nebeneinander und/oder übereinander angeordnet werden. Dabei können die Drehflügel auch an unterschiedlichen Behälterwänden vorgesehen werden.

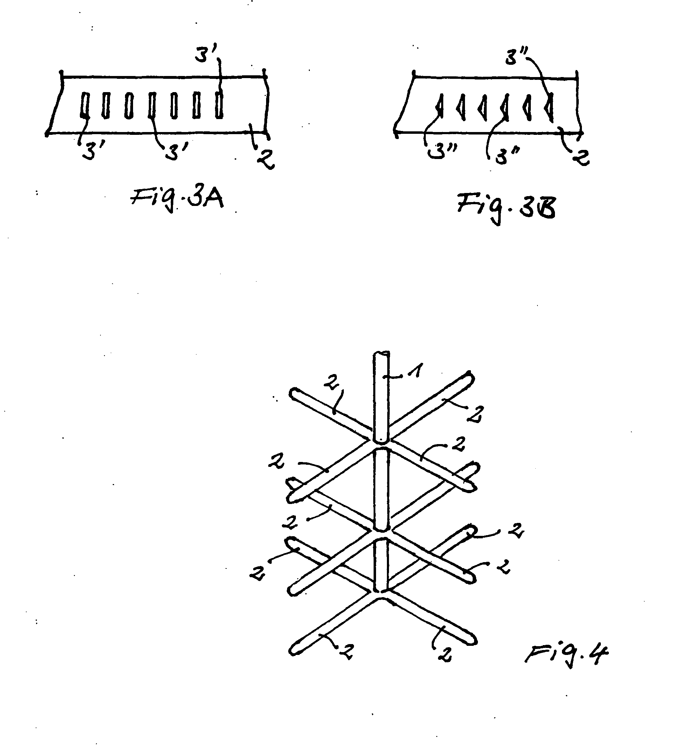

Gemäß den Figuren 3A und 3B kann der Flüssigkeitsstrahl auch in der Form von sogenannten Flüssigkeitsfächern erzeugt werden. Dies bedeutet, dass die Düsen 3', 3" nicht, wie bisher erläutert, einen kreisförmigen Querschnitt, sondern einen Querschnitt mit einer beliebigen geometrische Form, z.B. einen rechteckigen Querschnitt gemäß Figur 3A oder einen schlitzförmigen Querschnitt oder einen dreieckförmigen Querschnitt gemäß Figur 3B besitzen, wobei die Ausrichtung der Querschnitte zur Längsachse der Drehflügel 2 anwendungsspezifisch gestaltet werden kann.

Gemäß Figur 4 können beispielsweise an der hohlen Drehachse 1 auch mehrere Drehflügel 2. an axial voneinander beabstandeten Orten der Drehachse 1 ein Paar oder auch mehrere Paare von zwei sich radial gegenüberliegenden Drehflügeln 2 vorgesehen sein. Mehrere Paare sind dabei vorzugsweise gleichmäßig über den Umfang der Drehachse 1 verteilt.

Auf diese Weise kann, je nach der gestellten Aufgabe eine besonders effektive Reinigung erreicht werden.

Es wird darauf hingewiesen, dass die vorliegende Einrichtung nicht nur zur Reinigung von medizinischen Instrumenten, sondern allgemein auch zur Reinigung von beliebigen anderen Gegenständen geeignet ist.

## Patentansprüche

1. Einrichtung zur Reinigung von Gegenständen, insbesondere von medizinischen Instrumenten, mittels Ultraschall, mit einer Drehflingelanordnung, wobei die Drehflügelanordnung an einem Ort einer hohlen Drehachse (1) sich radial gegenüberliegend zwei hohle Drehflügel (2) umfasst, und jeder Drehflügel (2) wenigstens eine Düse (3) besitzt, **dadurch gekennzeichnet, dass** der wenigstens eine Ultraschallgenerator (5) Ultraschallwellen in einer Übertragungsflüssigkeit erzeugt und die Ultraschallwellen zu den zu reinigenden Gegenständen über eine Düse (3) der Drehflügelanordnung übertragen werden, und der Ultraschallgenerator (5) so angeordnet ist, dass er Ultraschallwellen in dem die Drehflügelanordnung durchströmenden Übertragungsmedium derart erzeugt, dass die die Düsen (3) der Drehflügel (2) verlassenden Flüssigkeitsstrahlen (10) von den Ultraschallwellen beaufschlagt sind.

2. Einrichtung nach Anspruch 1,
**dadurch gekennzeichnet, dass** wenigstens ein Ultraschallgenerator (5) in der hohlen Drehachse (1) angeordnet ist, wobei Reflektoren (7) vorgesehen sind, die die sich von der Sonotrode (5) aus in Richtung auf, die Drehflügel (2) ausbreitenden Ultraschallwellen in das Innere der Drehflügel (2) und aus dem Inneren der Drehflügel (2) zu den Düsen (3) lenken.

3. Einrichtung nach Anspruch 2, **dadurch gekennzeichnet, dass** die Reflektoren die Form von schräg im Inneren der Drehflügel (2) montierten Reflektorspiegeln aufweisen.

4. Einrichtung nach einem der Ansprüche 1 bis 3,
**dadurch gekennzeichnet, dass** wenigstens ein Ultraschallgenerator (5) im Inneren der Drehachse (1) angeordnet ist.

5. Einrichtung nach Anspruch 1, **dadurch gekennzeichnet, dass** im Inneren der Drehflügel (2) jeweils ein einer Düse (3) zugeordnete Ultraschallgenerator (51) vorgesehen ist, der das zu der ihm zugeordneten Düse (3) strömenden Übertragungsmedium mit Ultraschallwellen beaufschlagt.

6. Einrichtung nach einem des Ansprüche 1 bis 5, **dadurch gekennzeichnet, dass** an einem Ort der Drehachse (1) sich radial gegenüberliegend mehrere Paare von jeweils zwei Drehflügeln (2) angeordnet sind.

7. Einrichtung einem des Ansprüche 1 bis 6, **dadurch gekennzeichnet, dass** an der Drehachse (1) Drehflügel (2) axial voneinander beabstandet angeordnet sind.

8. Einrichtung nach Anspruch 7, **dadurch gekennzeichnet, dass** an axial voneinander beabstandeten Orten der Drehachse (1) sich jeweils radial gegenüberliegend zwei Drehflügel (2) angeordnet sind.

9. Einrichtung nach Anspruch 7, **dadurch gekennzeichnet, dass** an axial voneinander beabstandeten Orten der Drehachse (1) jeweils mehrere sich radial gegenüberliegende Paare von zwei Drehflügeln (2) angeordnet sind.

10. Einrichtung nach einem der Ansprüche 1 bis 7, **dadurch gekennzeichnet, dass** der oder die Drehflügel (2) jeweils mehrere zu einer oder mehreren Seiten des Drehflügels (2) weisende, mit dem Inneren des Drehflügels (2) in Verbindung stehende Düsen (3) aufweisen, wobei an der einen oder an den mehreren Seiten zu reinigende Gegenstände angeordnet sind.

11. Einrichtung nach einem der Ansprüche 1 bis 10, **dadurch gekennzeichnet, dass** die Düsen (3) in der Längsrichtung des Drehflügels (2) voneinander beabstandet vorgesehen sind.

12. Einrichtung nach einem der Ansprüche 1 bis 11, **dadurch gekennzeichnet, dass** die Düsen (3) in einem beliebigen Muster an dem Drehflügel (2) angeordnet sind.

13. Einrichtung nach einem der Ansprüche 1 bis 12,
**dadurch gekennzeichnet, dass** die Düsen (3) einen kreisförmigen Querschnitt besitzen.

14. Einrichtung nach einem der Ansprüche 1 bis 12,
**dadurch gekennzeichnet, dass** die Düsen (3) einen nicht kreisförmigen Querschnitt in einer beliebigen Form besitzen.

15. Einrichtung nach Anspruch 14, **dadurch gekennzeichnet, dass** die Düsen (3) einen rechteckigen, schlitzförmigen, dreieckförmigen oder ovalen Querschnitt besitzen.

16. Einrichtung nach einem der Ansprüche 1 bis 15,
**dadurch gekennzeichnet, dass** der Querschnitt der Düsen (3) in einer vorbestimmten Weise zur Längsachse des Drehflügels (2) ausgerichtet ist.

## Claims

1. A device for cleaning objects, especially medical instruments, by means of ultrasonic sound, comprising a rotary vane arrangement, with the rotary vane arrangement comprising two hollow rotary vanes (2) radially opposite of each other on a location of a hollow rotary shaft (1) and each of the vanes (2) comprising at least one nozzle (3), **characterized in that** the at least one ultrasonic generator (5) generates ultrasonic waves in a transmission liquid and the ultrasonic waves are transmitted to the objects to be cleaned via a nozzle (3) of the rotary vane arrangement, and the ultrasonic generator (5) is arranged in such a way that it generates ultrasonic waves in the transmission medium flowing through the rotary vane arrangement in such a way that the liquid jets (10) leaving the nozzles (3) of the rotary vanes (2) are subjected to ultrasonic waves.

2. A device according to claim 1, **characterized in that** at least one ultrasonic generator is arranged in the hollow rotary shaft (1), with reflectors being provided which guide the ultrasonic waves propagating from the sonotrode (5) in the direction of the vanes (2) into the interior of the vanes (2) and from the interior of the vanes (2) to the nozzles (3).

3. A device according to claim 2, **characterized in that** the reflectors have the shape of reflector mirrors mounted in an oblique fashion in the interior of the vanes (2).

4. A device according to one of the claims 1 to 3, **characterized in that** at least one ultrasonic generator (5) is arranged in the interior of the rotary shaft (1).

5. A device according to claim 1, **characterized in that** one ultrasonic generator (51) each which is associated with a nozzle (3) is provided in the interior of the vanes (2), which generator applies ultrasonic waves to the transmission medium flowing to the nozzle (3) associated with the same.

6. A device according to one of the claims 1 to 5, **characterized in that** several pairs of two vanes (2) each are arranged radially opposite of one another at one location of the rotary shaft (1).

7. A device according to one of the claims 1 to 6, **characterized in that** vanes (2) are arranged to be axially spaced from one another on the rotary shaft (1).

8. A device according to claim 7, **characterized in that** two vanes (2) each are arranged radially opposite of one another at locations of the rotary shaft (1) which are axially spaced from one another.

9. A device according to claim 7, **characterized in that** several pairs of two vanes (2) each are arranged radially opposite of one another at locations of the rotary shaft (1) which are axially spaced from one another.

10. A device according to one of the claims 1 to 7,
**characterized in that** the vane or vanes (2) each comprise several nozzles (3) facing towards one or several sides of the vane (2) and are in connection with the interior of the vane (2), with objects to be cleaned being arranged on the one side or the several sides.

11. A device according to one of the claims 1 to 10, **characterized in that** the nozzles (3) are provided to be spaced from one another in the longitudinal direction of the vane (2).

12. A device according to one of the claims 1 to 11,
**characterized in that** the nozzles (3) are arranged in any random pattern on the vane (2).

13. A device according to one of the claims 1 to 12,
**characterized in that** the nozzles (3) have a circular cross section.

14. A device according to one of the claims 1 to 12,
**characterized in that** the nozzles (3) have a non-circular cross section in a random shape.

15. A device according to claim 14, **characterized in that** the nozzles (3) have a rectangular, slit-like, triangular or oval cross section.

16. A device according to one of the claims 1 to 15,
**characterized in that** the cross section of the nozzles (3) is aligned in a predetermined manner in relation to the longitudinal axis of the vane (2).

## Revendications

1. Dispositif pour le nettoyage d'objets, en particulier d'instruments médicaux, au moyen d'ultrasons, avec un dispositif à ailettes tournantes, dans lequel le dispositif à ailettes tournantes comprend deux ailettes tournantes creuses (2) se faisant face dans le sens radial à un endroit d'un arbre de rotation creux (1) et chaque ailette tournante (2) possède au moins une buse (3) , **caractérisé en ce que** l'au moins un générateur d'ultrasons (5) produit des ondes ultrasonores dans un liquide de transmission et les ondes ultrasonores sont transmises aux objets à nettoyer via une buse (3) du dispositif à ailettes tournantes, et le générateur d'ultrasons (5) est disposé de telle façon qu'il produit des ondes ultrasonores dans le fluide de transmission parcourant le dispositif à ailettes tournantes, de telle façon que les jets de liquide (10) qui sortent des buses (3) des ailettes tournantes (2) soient soumis à l'action des ondes ultrasonores.

2. Dispositif selon la revendication 1, **caractérisé en ce qu'**au moins un générateur d'ultrasons (5) est disposé dans l'arbre de rotation creux (1), des réflecteurs (7) étant prévus pour guider les ondes ultrasonores se propageant à partir de la sonotrode (5) en direction des ailettes tournantes (2) vers l'intérieur des ailettes tournantes (2) et de l'intérieur des ailettes tournantes (2) vers les buses (3).

3. Dispositif selon la revendication 2, **caractérisé en ce que** les réflecteurs prennent la forme de miroirs réfléchissants montés à l'oblique à l'intérieur des ailettes tournantes (2).

4. Dispositif selon l'une des revendications 1 à 3, **caractérisé en ce qu'**au moins un générateur d'ultrasons (5) est disposé à l'intérieur de l'axe de rotation (1).

5. Dispositif selon la revendication 1, **caractérisé en ce qu'**il est prévu à l'intérieur des ailettes tournantes (2) un générateur d'ultrasons (51) pour chaque buse (3), qui soumet le fluide de transmission circulant vers la buse (3) qui lui est associée à l'action d'ondes ultrasonores.

6. Dispositif selon l'une des revendications 1 à 5, **caractérisé en ce que** plusieurs paires de deux ailettes tournantes (2) se faisant face dans le sens radial sont disposées à un endroit de l'axe de rotation (1).

7. Dispositif selon l'une des revendication s 1 à 6, **caractérisé en ce que** des ailettes tournantes (2) sont disposées à distance les unes des autres dans le sens axial sur l'axe de rotation (1).

8. Dispositif selon la revendication 7, **caractérisé en ce que** deux ailettes tournantes (2) se faisant face dans le sens radial sont disposées à des endroits de l'axe de rotation (1) distants dans le sens axial.

9. Dispositif selon la revendication 7, **caractérisé en ce que** plusieurs paires d'ailettes tournantes (2) se faisant face dans le sens radial sont disposées à plusieurs endroits de l'axe de rotation (1) distants dans le sens axial.

10. Dispositif selon l'une des revendications 1 à 7, **caractérisé en ce que** la ou les ailettes tournantes (2) présentent chacune plusieurs buses (3) dirigées vers une ou plusieurs faces des ailettes tournantes (2) et communiquant avec l'intérieur des ailettes tournantes (2), des objets à nettoyer étant disposés sur la face ou les plusieurs faces.

11. Dispositif selon l'une des revendications 1 à 10, **caractérisé en ce que** les buses (3) sont disposées à distance les unes des autres dans le sens longitudinal des ailettes tournantes (2).

12. Dispositif selon l'une des revendications 1 à 11, **caractérisé en ce que** les buses (3) sont disposées selon un schéma quelconque sur les ailettes tournantes (2).

13. Dispositif selon l'une des revendications 1 à 12, **caractérisé en ce que** les buses (3) ont une section circulaire.

14. Dispositif selon l'une des revendications 1 à 12, **caractérisé en ce que** les buses (3) ont une section non circulaire d'une forme quelconque.

15. Dispositif selon la revendication 14, **caractérisé en ce que** les buses (3) ont une section rectangulaire, en forme de fente, triangulaire ou ovale.

16. Dispositif selon l'une des revendications 1 à 15, **caractérisé en ce que** la section des buses (3) est orientée de manière prédéterminée vers l'axe longitudinal des ailettes tournantes (2).
